(19) **Europäisches Patentamt / European Patent Office / Office européen des brevets**

(11) **EP 3 890 533 B1**

(12) **FASCICULE DE BREVET EUROPEEN**

(45) Date de publication et mention de la délivrance du brevet:
**14.02.2024 Bulletin 2024/07**

(21) Numéro de dépôt: **19868219.7**

(22) Date de dépôt: **06.12.2019**

(51) Classification Internationale des Brevets (IPC):
**A41B 11/12** *(2006.01)* **A61F 13/08** *(2006.01)*

(52) Classification Coopérative des Brevets (CPC):
**A61F 13/08; A41B 11/126;** A41D 2400/80

(86) Numéro de dépôt international:
**PCT/FR2019/052949**

(87) Numéro de publication internationale:
**WO 2020/115446 (11.06.2020 Gazette 2020/24)**

(54) **DISPOSITIF DE RETENUE ET SYSTÈME DE CONTENTION COMPRENANT UN ARTICLE DE CONTENTION ET UN TEL DISPOSITIF DE RETENUE**

HALTEVORRICHTUNG SOWIE RÜCKHALTESYSTEM MIT EINER RÜCKHALTEBEKLEIDUNG UND SOLCH EINER HALTEVORRICHTUNG

HOLDING DEVICE AND RETENTION SYSTEM COMPRISING A RETENTION GARMENT AND SUCH A HOLDING DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.12.2018 FR 1872513**

(43) Date de publication de la demande:
**13.10.2021 Bulletin 2021/41**

(73) Titulaire: **URGO RECHERCHE INNOVATION ET DEVELOPPEMENT**
**21300 Chenôve (FR)**

(72) Inventeur: **LECOMTE, Serge**
**21000 DIJON (FR)**

(74) Mandataire: **Plasseraud IP**
**66, rue de la Chaussée d'Antin**
**75440 Paris Cedex 09 (FR)**

(56) Documents cités:
**EP-A2- 2 810 571        WO-A1-93/04858**
**WO-A1-96/12844        DE-A1-102006 022 971**
**GB-A- 2 527 730**

Processed by Luminess, 75001 PARIS (FR)

## Description

### Domaine technique

L'invention se rapporte à un dispositif de retenue et à un système de contention comprenant un article de contention et un tel dispositif de retenue.

L'invention se rapporte, en particulier, à un dispositif de retenue destiné à prévenir un glissement d'un article de contention sur un membre d'un individu.

### Technique antérieure

Sans y être limitée, l'invention s'applique tout particulièrement à un article de contention de type textile, pouvant présenter des boucles en surface, telle qu'une bande de contention textile et notamment une bande de contention à allongement court.

Des articles de contention de type textile, enroulés ou enfilés sur un membre, en extension pour appliquer une pression, sont couramment utilisés pour le traitement de diverses pathologies comme par exemple les problèmes de circulation veineuse, les ulcères de jambe ou le traitement des lymphoedèmes.

Un problème récurrent avec ces articles de contention est, quand le traitement s'applique aux membres inférieurs, leur glissement au cours du temps le long de la jambe à cause de leurs poids et du mouvement des patients. Ce phénomène est le plus critique dans le cas des bandes de contention, que l'on enroule autour de la jambe jusque sous le genou, par exemple dans le traitement des ulcères de jambe. En effet, la bande de contention qui recouvre le mollet est soumise à des pressions élevées à appliquer et, lors de la marche, à des variations rapides et répétitives du diamètre du mollet. La bande de contention est alors soumise à des contraintes mécaniques importantes qui entraînent son glissement le long de la jambe. Ce glissement peut notamment résulter d'un relâchement intrinsèque des spires par rapport à une tension exercée lors de la pose.

Les bandes de contention sont souvent classées en allongement long ou court. Cette classification est basée sur la mesure de l'allongement longitudinal de la bande de contention telle que définie dans la méthode A §9.1 de la norme EN 14704-1 quand la bande de contention est soumise à une force de traction maximale de 6 N/cm.

Les conditions de réalisation de la mesure sont les suivantes. Une éprouvette de la bande de contention à tester de 50 mm de largueur et de 250 mm à 300 mm de longueur est découpée et positionnée sans précontrainte dans des mors d'un dynamomètre électronique (par exemple un dynamomètre de marque MTS) de sorte à avoir une largeur de 50 mm et une longueur utile de référence de 200 mm. Une traverse du dynamomètre étire l'éprouvette depuis une position initiale à une vitesse de 100 mm/min jusqu'à une force maximale de 6 N/cm puis revient à sa position initiale à la même vitesse de retour de 100 mm/min. Ce cycle est effectué 5 fois et l'allongement obtenu au cinquième cycle, exprimé en pourcentage, est directement calculé par le dynamomètre. L'opération est répétée sur 5 éprouvettes, puis on calcule la valeur moyenne qui définit l'allongement longitudinal de la bande.

Sur la base de ce test selon la norme EN 14704-1 pris comme référence, on considère qu'une bande de contention est une bande à « allongement court » si son allongement longitudinal est inférieur ou égal à 100 % et une bande à « allongement long » si son allongement longitudinal est supérieur à 100 %.

Il est reconnu aujourd'hui que les systèmes de contention les plus performants en termes de facilité et de rapidité de pose et en termes d'efficacité thérapeutique sont ceux qui comprennent au maximum 2 bandes et au moins une bande de contention dite à allongement court. Ces bandes de contention exercent une pression au repos basse et une pression de travail élevée. Elles ont donc un grand différentiel de pression en particulier lors des mouvements, par exemple pendant la marche.

Mais en contrepartie ces bandes à allongements courts sont celles qui présentent le plus fort potentiel de glissement.

Pour éviter le glissement des articles de contention, deux grandes solutions techniques ont été envisagées.

La première solution technique consiste à augmenter le coefficient de friction entre l'article de contention textile et la peau.

Pour cela, la rugosité de tout ou partie de la face du textile venant en contact avec la peau est augmentée par incorporation de fils ou de bandes présentant un caractère plus rugueux ou par la création de surfaces non lisses. Des exemples mettant en oeuvre une telle solution sont par exemple décrits dans les documents WO 2012/140558, WO 2015/94385, WO 2016/177829 ou US 6 194 629.

Cette première solution technique est privilégiée pour les bas et chaussettes. En revanche, elle n'est pas utilisée dans le cas des bandes de contention où les contraintes mécaniques sont plus importantes. De plus, elle complexifie la mise au point et la fabrication de l'article de contention et l'augmentation de la friction avec la peau conduit à des articles de contention qui peuvent être moins bien acceptés par les patients, en particulier si la peau est fragile ou lésée comme dans le traitement des ulcères de jambe.

La seconde solution technique consiste à déposer sur tout ou partie de l'article de contention textile un adhésif pour coller sur la peau. Une telle solution est par exemple décrite dans WO 2009/138672 pour les bas. D'autres solutions sont divulguées dans les documents EP281057 ou DE102006022971.

Cette solution complexifie aussi la réalisation et la fabrication de l'article de contention. Il faut que l'adhésif s'ancre bien dans le matériau textile, résiste aux contraintes mécaniques lors des mouvements et ne modifie pas après son application sur le textile les propriétés mécaniques de ce dernier, telle l'élasticité et l'extensibilité. Elle est au final peu employée, en particulier si l'on souhaite laver ultérieurement l'article de contention pour le réutiliser. De façon générale la présence d'adhésif sur l'article de contention est souvent rejetée par les utilisateurs.

Enfin, dans le cas d'une bande de contention, la position de l'adhésif sur seulement une partie de la bande est insoluble sauf à développer une multitude de bandes de contention pour s'adapter à la morphologie de chaque patient dont les jambes sont plus ou moins longues et la position de la plaie variable sur la jambe. De plus, dans le cas du traitement des ulcères de jambe, on place sur la plaie un pansement que la bande de contention vient recouvrir et il faut éviter que la bande de contention ne colle au pansement.

Dans le domaine des bandes de contention, pour garantir le maintien, les deux côtés de la bande de contention sont généralement enduits avec un produit, tel que du latex, qui n'adhère pas à la peau mais uniquement sur lui-même. Ce dernier bloque l'enroulement de la bande de contention en extension et évite le glissement spire sur spire, son relâchement et son glissement.

Mais à nouveau, la réalisation d'une telle bande de contention auto adhérente, en particulier à allongement court, est complexe car l'enduction du latex modifie les propriétés d'élasticité et d'extensibilité de la bande de contention et risque de diminuer sa respirabilité. Pour simplifier la mise au point, dans les articles de contention les plus récents, la pression a été répartie sur deux bandes de contention. La deuxième bande de contention permet de fixer et maintenir la première bande de contention. Mais dans ce cas, des produits plus épais, plus chauds et surtout plus longs à poser sont obtenus. Enfin, le latex est susceptible de causer des allergies chez certains patients.

Le rôle d'un adhésif ou du latex a longtemps été incontournable pour maintenir la bande de contention ou le système de contention après son enroulement autour d'un membre et diminuer leur relâchement intrinsèque qui conduit à leur perte d'efficacité et leur glissement au cours du temps le long du membre.

Afin d'améliorer l'acceptabilité par les patients et le personnel soignant et d'obtenir un système de contention plus facile à fabriquer, il apparaît donc souhaitable de disposer d'un article de contention qui utilise des bandes de contention sans adhésif ou latex. Un premier avantage est de permettre de laver et réutiliser la bande de contention. Un deuxième avantage est, dans le cas des systèmes de contention multi couches, d'obtenir des produits moins épais, moins chauds et surtout plus rapides à poser du fait de la suppression de la couche auto adhérente.

La demande WO 2017/089731 propose un tricot 3D qui présente une contrainte de seuil de cisaillement supérieure ou égale à 2800 Pa pour éviter le glissement spire sur spire qui induit le glissement final de la bande de contention. Mais cette solution spécifique n'est pas applicable à d'autres bandes de contention ou à des tricots 3D dont la contrainte de cisaillement est inférieure à 2800Pa.

Il est donc souhaitable de trouver une solution plus universelle pour éviter le glissement d'une bande de contention non auto adhérente et plus généralement de tout article de contention susceptible de glisser le long de la jambe.

L'invention vise à proposer une telle solution.

## Exposé de l'invention

A cette fin, selon un premier aspect, l'invention propose un dispositif de retenue destiné à prévenir un glissement d'un article de contention sur un membre d'un individu selon la revendication 1. Le dispositif de retenue comprenant :

- un film souple et inextensible présentant une première face destinée à être placée en contact avec l'article de contention, et une deuxième face opposée à la première face et destinée à être placée en regard du membre de l'individu, la première face comportant une surface de base et une pluralité de saillies s'étendant chacune depuis la surface de base jusqu'à un bord libre, chaque saillie présentant une hauteur entre la surface de base et le bord libre comprise entre 80 $\mu$m et 1200 $\mu$m, de préférence entre 150 $\mu$m et 900 $\mu$m, et étant conformée en une paroi dont le bord libre a une longueur, les saillies étant agencées sur la surface de base de telle manière qu'une somme des longueurs des bords libres par unité de surface de la surface de base soit comprise entre 5 cm/cm$^2$ et 120 cm/cm$^2$, de préférence entre 10 cm/cm$^2$ et 80 cm/cm$^2$,
- une couche adhésive recouvrant au moins en partie la deuxième face du film et adaptée pour retenir le film sur le membre de l'individu.

Le déposant a déterminé que, parmi l'ensemble des forces qui interagissent sur un article de contention adhésivé et posé en extension, le point le plus critique est la liaison entre l'adhésif et le matériau textile de l'article de contention. Le matériau textile est extensible de sorte que les contraintes de cisaillement que subit l'article de contention sont directement transmises à l'adhésif. Dans le cas d'une bande de contention, ces contraintes sont très importantes et la liaison entre l'adhésif et le matériau textile devient alors très fragile.

L'invention permet de favoriser le lien entre l'adhésif et l'article adhésif même lors des variations d'extensibilité de ce dernier. Le déposant a déterminé que pour protéger cette liaison, il faut inhiber ou au moins diminuer la transmission de la contrainte de cisaillement qu'impose l'article de contention lors des mouvements à l'adhésif. Pour obtenir ce résultat, il a interposé entre une couche adhésive et l'article de contention, un film qui, grâce à son inextensibilité et sa capacité de friction résultant de la rugosité de la première face, s'oppose à la contrainte de cisaillement.

Ainsi, l'invention propose un dispositif de retenue anti-glissement d'un article de contention posé en extension, en particulier sur la jambe. Le dispositif de retenue inhibe efficacement et durablement le glissement spire sur spire et le glissement global, en particulier dans le cas d'une bande de contention à allongement court non auto-adhérente. Le dispositif de retenue est indépendant de l'article de contention de manière à permettre une réutilisation de cet article de contention.

En outre, le dispositif de retenue est facile à utiliser et offre un plus grand choix de substance d'adhésive. L'invention propose une solution plus universelle et plus simple à mettre en oeuvre que celles décrites dans les documents WO 2017/089731 et WO 2009/138672. En particulier, dans le document WO 2009/138642 où le matériau textile est directement enduit, il est prévu une sélection spécifique d'un adhésif qui doit présenter un module élastique au moins aussi bas que celui du matériau sur lequel il est appliqué pour ancrer l'adhésif dans le matériau mais éviter qu'il ne migre trop à l'intérieur du textile et modifie ses propriétés d'extensibilité et d'élasticité.

Le dispositif peut comprendre les éléments des revendications 2 à 14. Le dispositif peut comprendre les éléments suivants :

- La paroi de chaque saillie s'étend autour d'un axe central sensiblement normal à la surface de base, la longueur du bord libre de chaque saillie définissant un périmètre.

- Les saillies sont formées en une seule pièce avec la surface de base.

- Le film comporte une pluralité de perforations réalisées au travers du film depuis la deuxième face de manière à obtenir des déformations locales du film sur la première face, chaque déformation locale entourant l'une des perforations et formant la paroi de l'une des saillies.

- Les saillies sont agencées selon un réseau ayant une densité comprise entre 15 saillies/cm$^2$ et 700 saillies/cm$^2$, de préférence entre 80 saillies/cm$^2$ et 300 saillies/cm$^2$.

- La deuxième face du film présente une surface lisse.

- Le film est réalisé en polymère thermoplastique, notamment en polymère à base de polyoléfines, tels que du polyéthylène, en copolymère de polyéthylène et d'acétate de vinyle, en copolymère de polyéthylène et d'acrylate de butyle ou de polychlorure de vinyle plastifié, en polymère ou copolymère de polyester, en polymère ou copolymère de polyamide, en polymère ou copolymère de polyuréthanne ou en polymère de polycarbonate.

- Le film a un grammage compris entre 10 g/m$^2$ et 120 g/m$^2$, de préférence entre 20 g/m$^2$ et 60 g/m$^2$.

- La couche adhésive présente une résistance t au cisaillement tangentiel d'au moins 10 heures selon un test de fluage dans lequel une première partie du dispositif de retenue est collée par l'intermédiaire de la couche adhésive à une plaque inclinée d'un angle de 2°, par rapport à une direction verticale, en laissant libre une deuxième partie du dispositif de retenue, un lest étant suspendu à la deuxième partie du dispositif de retenue, avec : t = (L$^2$.l. η) / 2.e.M.g où L est une longueur de la couche adhésive de la première partie du dispositif de retenue,

  l est une largeur de la couche adhésive de la première partie du dispositif de retenue,
  η est une viscosité de la couche adhésive,
  e est une épaisseur de la couche adhésive,
  M est une masse du lest, M étant choisie en fonction d'une contrainte tangentielle exercée par l'article de contention,

$$g = 9{,}81 \ m/s^2.$$

- La couche adhésive est constituée d'une substance adhésive sensible à la pression et notamment d'une substance adhésive choisie parmi :

- les adhésifs acryliques à base de polymères acrylates ou de copolymères d'acide acrylique et d'esters de l'acide acrylique,
- les adhésifs à base de poly-isobutylène de haut poids moléculaires,
- les adhésifs à base de poly-isoprène naturel ou synthétique,
- les adhésifs à base de polymères ou copolymères de siloxane et de résines tackifiantes ou les gels de silicone à base de polyxilosanes réticulés,
- les adhésifs à base de polyuréthanne ou les gels de polyuréthanne,
- les adhésifs à base de copolymères triblocs, de plastifiants et de résines tackifiantes, en particulier les polymères triblocs du type styrène-séquence centrale-styrène dans laquelle la séquence centrale est insaturée, par exemple isoprène ou butadiène, ou saturée, par exemple éthylène-butylène ou éthylène-propylène, éventuellement mélangés avec des copolymères diblocs comme par exemple des copolymères styrène-isoprène ou styrène-butadiène.

- La couche adhésive comporte une unique couche adhésive élémentaire.

- La couche adhésive élémentaire a un grammage d'au moins 50 g/m$^2$, de préférence compris entre 60 g/m$^2$ et 120 g/m$^2$.

- La couche adhésive élémentaire a une force d'adhérence comprise entre 0,5 N/cm et 5 N/cm mesurée par un test de pelage dans lequel la force d'adhérence selon un angle de 90° pour décoller la couche adhésive d'une surface de référence choisie parmi la deuxième face du film et une surface de peau est mesurée.

- La couche adhésive comprend :

  - une première couche adhésive élémentaire destinée à être mise en contact avec le membre,
  - une âme présentant une première surface portant la première couche adhésive élémentaire, et une deuxième surface opposée à la première surface,
  - une deuxième couche adhésive élémentaire pour fixer la deuxième surface de l'âme à la deuxième face du film.

- La première couche adhésive élémentaire a un grammage compris entre 25 g/m$^2$ et 120 g/m$^2$, de préférence entre 40 g/m$^2$ et 80 g/m$^2$, et la deuxième couche adhésive élémentaire peut avoir un grammage compris entre 20 g/m$^2$ et 120 g/m$^2$, de préférence entre 30 g/m$^2$ et 50 g/m$^2$.

- La première couche adhésive élémentaire a une force d'adhérence comprise entre 0,5 N/cm et 5 N/cm et la deuxième couche adhésive élémentaire peut avoir une force d'adhérence comprise entre 0,5 N/cm et 10 N/cm, la force d'adhérence de chacune des couches adhésives élémentaires choisie parmi les première et deuxième couches adhésives élémentaires étant mesurée par un test de pelage dans lequel la force d'adhérence selon un angle de 90° pour décoller ladite couche adhésive élémentaire d'une surface de référence choisie parmi la deuxième face du film et une surface de peau est mesurée.

Selon un deuxième aspect, l'invention propose un système de contention défini dans la revendication 15 et comprenant un article de contention destiné à être placé sur un membre d'un patient et un dispositif de retenue tel que défini précédemment.
L'article de contention peut être une bande de contention textile destinée à être enroulée sur le membre.
La bande de contention peut être à allongement court.

**Brève description des dessins**

D'autres caractéristiques, détails et avantages de l'invention apparaîtront à la lecture de la description détaillée ci-après, et à l'analyse des dessins annexés, sur lesquels :

[Fig. 1] - la figure 1 est une représentation d'un dispositif de retenue selon un mode de réalisation de l'invention, le dispositif de retenue comprenant un film dont une première face comporte une pluralité de saillies et une deuxième face est recouverte au moins en partie d'une couche adhésive,

[Fig. 2] - la figure 2 est une représentation schématique agrandie en coupe selon l'orientation référencée II-II sur la figure 1 de l'une des saillies du dispositif de retenue de la figure 1, la saillie comprenant une paroi autour d'un axe central formée par une déformation locale du film obtenue à partir d'une perforation réalisée depuis la deuxième face, la couche adhésive comprenant une unique couche adhésive élémentaire,

[Fig. 3] - la figure 3 est une représentation schématique de la première face du film de la figure 1 illustrant une méthode de calcul d'une somme de longueurs de bords libres des saillies par unité de surface, laquelle somme est comprise entre 5 cm/cm$^2$ et 120 cm/cm$^2$,

[Fig. 4] - la figure 4 est une représentation schématique agrandie en coupe selon l'orientation référencée II-II sur la figure 1 de l'une des saillies d'une variante du dispositif de retenue de la figure 1, la couche adhésive comprenant des première et deuxième couches adhésives élémentaires entre lesquelles une âme est interposée,

[Fig. 5] - la figure 5 est une représentation d'un dispositif expérimental pour réaliser un test de fluage mesurant une résistance au cisaillement tangentiel de la couche adhésive,

[Fig. 6] - la figure 6 est une représentation d'un test in vivo mettant en oeuvre un article de contention sous la forme d'une bande de contention à allongement court enroulée sur la jambe d'un individu, illustrant un glissement de la bande de contention,

[Fig. 7] - la figure 7 est une représentation d'un test in vivo mettant en oeuvre un système de contention selon un mode de réalisation de l'invention comprenant le dispositif de retenue et un article de contention sous la forme d'une bande de contention à allongement court enroulée sur la jambe d'un individu, illustrant un glissement moindre de la bande de contention par rapport à la bande de contention enroulée sur la figure 6.

## Description des modes de réalisation

Sur les figures, les mêmes références désignent des éléments identiques ou analogues.

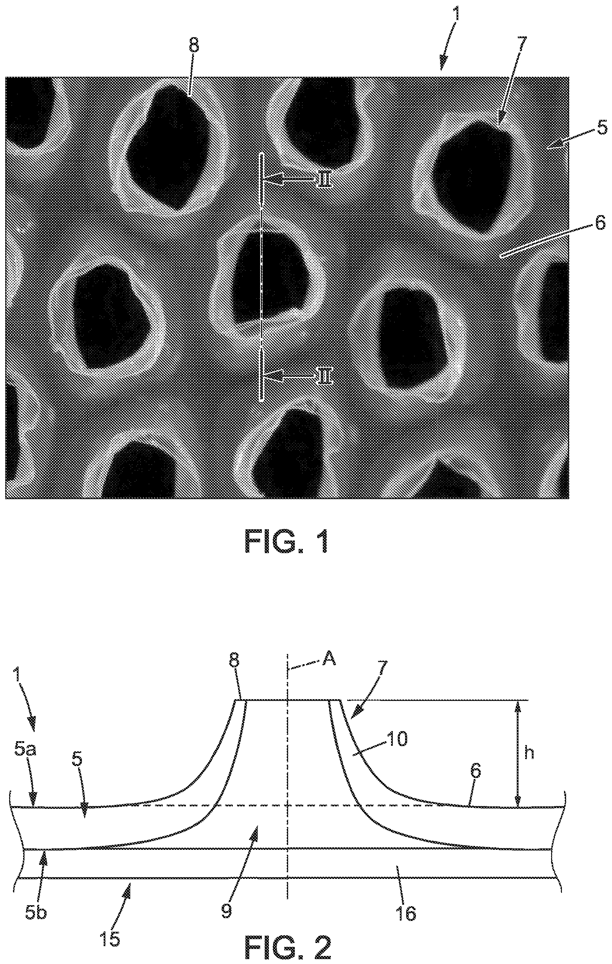

Les figures 1 et 2 représentent un dispositif de retenue 1 destiné à prévenir un glissement d'un article de contention sur un membre d'un individu. L'article de contention peut notamment être une bande de contention 2 en matériau textile, à allongement court comme défini dans la méthode A §9.1 de la norme EN 14704-1, enroulée sur une jambe 3 de l'individu.

Le dispositif de retenue 1 comprend :

- un film 5 souple et inextensible présentant une première face 5a destinée à être placée en contact avec l'article de contention, et une deuxième face 5b opposée à la première face 5a et destinée à être placée en regard du membre de l'individu, et
- une couche adhésive 15 recouvrant au moins en partie la deuxième face 5b du film 5 et adaptée pour retenir le film 5 sur le membre de l'individu.

En particulier, comme il ressort des figures 1 et 2, la première face 5a comporte une surface de base 6 et une pluralité de saillies 7 s'étendant chacune depuis la surface de base 6 jusqu'à un bord libre 8. Dans le mode de réalisation représenté, les saillies 7 sont formées en une seule pièce avec la surface de base 6 à partir d'une pluralité de perforations 9 réalisées au travers du film 5 depuis la deuxième face 5b de manière à obtenir des déformations locales du film 5 sur la première face 5a. Chaque déformation locale entoure l'une des perforations 9 en formant une paroi 10 de l'une des saillies 7. La paroi 10 de chaque saillie 7 s'étend alors autour d'un axe central A sensiblement normal à la surface de base 6. Le bord libre 8 de chaque saillie 7 a une longueur définissant, dans le mode de réalisation représenté, un périmètre.

L'invention n'est pas limitée à une telle réalisation et une telle conformation des saillies 7. L'invention s'étend à toute saillie 7 formée en une seule pièce avec la surface de base 6 ou rapportée sur la surface de base 6, et conformée en une paroi 10 fermée ou non, présentant un bord libre 8 continu ou discontinue ayant une longueur.

La deuxième face 5b du film 5 présente une surface lisse.

Le film 5 peut être monocouche ou multicouche. Il est, de préférence, réalisé à base de polymères thermoplastiques. Il est, par exemple, réalisé en polymère à base de polyoléfines, tels le polyéthylène (basse, moyenne ou haute densité), en copolymère de polyéthylène et d'acétate de vinyle, en copolymère de polyéthylène et d'acrylate de butyle ou de polychlorure de vinyle plastifié, en polymère ou copolymère de polyester, en polymère ou copolymère de de polyamide (6 ou 6.6), en polymère ou copolymère de polyuréthanne ou en polymère de polycarbonate.

Le film 5 peut être translucide ou transparent, éventuellement colorés. Les polymères peuvent ainsi être associés à des charges, comme par exemple le dioxyde de titane pour obtenir une couleur blanche.

Le film 5 a, de préférence, un faible grammage, c'est-à-dire de l'ordre de 10 g/m$^2$ à 100g/m$^2$, notamment entre

20 g/m$^2$ et 60 g/m$^2$. De plus, les performations 9 et les saillies 7 qui en résultent sont agencées selon un réseau ayant une densité comprise entre 15 saillies/cm$^2$ et 700 saillies/cm$^2$, notamment entre 80 saillies/cm$^2$ et 300 saillies/cm$^2$. Ces dispositions permettent d'améliorer la souplesse et la conformabilité du film 5. Le film 5 est alors non agressif et bien adaptable à la morphologie de la jambe 3 et il assure un excellent contact avec la bande de contention 2.

A titre d'exemple non limitatif, le film 5 est disponible à partir de films commercialisés par les sociétés TREDEGAR et RKW.

Les saillies 7 de la première face 5a du film 5 procurent un effet de friction sur le matériau textile de l'article de contention. Toutefois, pour assurer une meilleure efficacité en friction, la paroi 10 de chaque saillie 7 présente une hauteur h entre la surface de base 6 et le bord libre 8 comprise entre 80 μm et 1200 μm, de préférence entre 150 μm et 900 μm, et les saillies 7 sont agencées sur la surface de base 6 de telle manière qu'une somme des longueurs des bords libres 8 par unité de surface de la surface de base 6 soit comprise entre 5 cm/cm$^2$ et 120 cm/cm$^2$, de préférence entre 10 cm/cm$^2$ et 80 cm/cm$^2$.

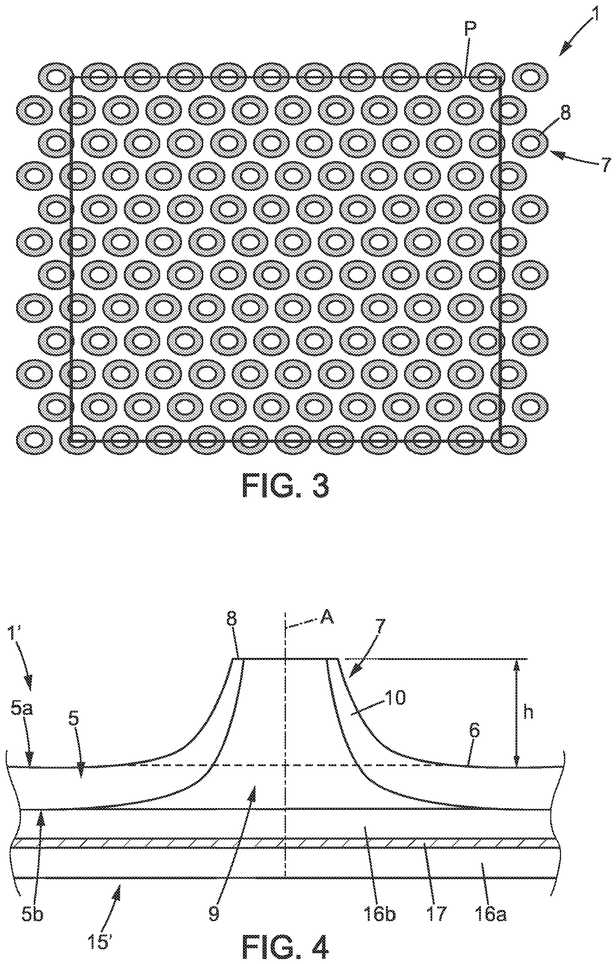

Comme représenté sur les figures 2 et 3, la hauteur h et la somme des longueurs de bords libres 8 des parois 10 des saillies 7 peuvent aisément être déterminées à partir de clichés microscopiques en mesurant la hauteur h et en quantifiant un nombre de saillies 7, dont la géométrie notamment cylindrique de section circulaire ou polygonale, est déterminée, ou portions de saillies sur l'unité de surface P.

Grâce à l'utilisation d'un tel film 5, une plus large palette de substances adhésives pour la couche adhésive 15 peut être utilisée dans le cadre de la réalisation du dispositif de retenue 1. De plus, vu l'orientation des saillies sur une seule face, la couche adhésive 15 peut être appliquée sur la deuxième face 5b lisse par transfert de façon à éviter une migration de substance adhésive dans l'article de contention.

Dans le cadre de la présente invention, la substance adhésive est choisie pour adhérer à la peau et au film. La substance adhésive est notamment une substance adhésive biocompatible couramment utilisée pour adhérer sur la peau ou une plaie dans des dispositifs médicaux tels des fixations médicales, des patchs ou des pansements et des produits cosmétiques.

La couche adhésive 15 est constituée d'une substance adhésive sensible à la pression désignée sous le terme de PSA pour *Pressure Sensitive Adhesive.* Une telle substance adhésive présente selon le PSTC (*Pressure Sensitive Tape Council*) les caractéristiques suivantes :

- un pouvoir collant permanent et mesurable,
- un pouvoir collant avec une pression à peine supérieure à celle du doigt
- une adhérence sans aucune activation d'une quelconque source d'énergie,
- une capacité suffisante à tenir sur le substrat,
- une cohésion suffisante pour se retirer du substrat sans résidus.

La substance adhésive peut notamment être choisie parmi :

- les adhésifs acryliques à base de polymères acrylates ou de copolymères d'acide acrylique et d'esters de l'acide acrylique ; ces adhésifs se présentent en phase aqueuse ou dispersion aqueuse, ils peuvent aussi être obtenus par photo polymérisation de monomères acryliques avec des photos amorceurs par exemple par réticulation UV,
- les adhésifs à base de poly-isobutylène de haut poids moléculaires,
- les adhésifs à base de poly-isoprène naturel ou synthétique,
- les adhésifs à base de polymères ou copolymères de siloxane et de résines tackifiantes ou les gels de silicone à base de polyxilosanes réticulés tels ceux décrits dans le document WO 2009/138642,
- les adhésifs à base de polyuréthanne ou les gels de polyuréthanne,
- les adhésifs à base de copolymères triblocs, de plastifiants et de résines tackifiantes, en particulier les polymères triblocs du type styrène-séquence centrale-styrène dans lequel la séquence centrale est insaturée, par exemple isoprène ou butadiène, ou saturée, par exemple éthylène-butylène ou éthylène-propylène, éventuellement mélangés avec des copolymères diblocs comme par exemple des copolymères styrène-isoprène ou styrène-butadiène.

Diverses charges peuvent aussi être incorporées dans la formulation de ces adhésifs comme par exemple des antioxydants, des colorants comme le dioxyde de titane ou des corps gras comme la vaseline ou la lanoline.

Ces substances adhésives sensibles à la pression sont en général hydrophobes mais peuvent incorporer si nécessaire des composés hydrophiles, comme par exemple des hydrocolloïdes comme le sel de sodium de la carboxyméthylcellulose, en quantité de l'ordre de 1 % à 40 % et, de préférence, de 2 % à 25 %.

A titre d'example non-limitatif, la substance adhésive est un adhésif à base de copolymères triblocs du type styrène-séquence centrale-styrène, de plastifiants, de résines tackifiantes et éventuellement de charges sur la peau normale. Pour les peaux fragiles, la formulation précitée sera complétée avec, en dispersion dans l'adhésif, une faible quantité d'hydrocolloïdes de 2 % à 20 % par rapport au poids total de l'adhésif ou des gels de silicone ou de polyuréthanne.

Si nécessaire, un traitement, notamment corona, peut être prévu sur le film 5 ou la couche adhésive 15 pour faciliter l'adhésion de la couche adhésive 15 sur le film 5.

Sur la figure 2, la couche adhésive 15 comporte une unique couche adhésive élémentaire 16.

Le grammage de la couche adhésive élémentaire 16 déposée sur la deuxième face 5b lisse du film 5 est, de préférence, d'au moins 50 g/m$^2$ et notamment compris entre 60 g/m$^2$ et 120 g/m$^2$, afin de coller à la fois à la peau et au film.

Un pouvoir adhésif de la couche adhésive 15 est défini par un test de pelage. Ce type de test est classique et décrit, par exemple, dans la norme EN 1939. Il consiste à mesurer une force d'adhérence à fournir pour décoller une couche adhésive 15 selon un angle de 90° d'une surface de référence. Ici, la force d'adhérence pour décoller la couche adhésive 15 de la deuxième face 5b du film 5 ou d'une surface de peau (par exemple sur l'avant-bras).

La force d'adhérence de la couche adhésive élémentaire 16 sur la peau peut être comprise entre 0,5 N/cm et 5 N/cm. Selon l'état de la peau (normale, altérée, fragile, paramètres souvent liés à l'âge du patient et à son état de santé), la morphologie de la jambe (musclée, graisseuse, conique ou osseuse) et l'activité physique du patient (mobile ou non), la couche adhésive élémentaire 16 avec le pouvoir adhésif le mieux adapté est choisie. Dans le cas d'une peau anormale, une couche adhésive dont la force d'adhérence est comprise entre 0,5 N/cm et 1,5 N/cm est préférée.

Selon une autre variante représentée sur la figure 4, en cas de difficultés pour adhérer à la fois à la peau et au film, le dispositif de retenue 1' peut avoir une couche adhésive 15' de type double face comprenant :

- une première couche adhésive élémentaire 16a destinée à être mise en contact avec le membre,
- une âme 17 présentant une première surface portant la première couche adhésive élémentaire 16a, et une deuxième surface opposée à la première surface,
- une deuxième couche adhésive élémentaire 16b pour fixer la deuxième surface de l'âme 17 à la deuxième face 5b du film 5.

L'âme est en général constituée d'un film et/ou un matériau textile. On préférera l'utilisation comme âme d'un film à base de polymère thermoplastique, d'un tricot, d'un tissé ou d'un non tissé ou leur association.

La première couche adhésive élémentaire 16a peut alors avoir un grammage compris entre 25 g/m$^2$ et 120 g/m$^2$, de préférence entre 40 g/m$^2$ et 80 g/m$^2$, et une force d'adhérence comprise entre 0,5 N/cm et 5 N/cm, notamment entre 0,5 N/cm et 1,5 N/cm pour une peau fragile.

La deuxième couche adhésive élémentaire 16b peut avoir un grammage compris entre 20 g/m$^2$ et 120 g/m$^2$, de préférence entre 30 g/m$^2$ et 50 g/m$^2$ et une force d'adhérence comprise entre 0,5 N/cm et 10 N/cm.

Dans le cas d'une bande de contention à allongement court, malgré la diminution ainsi obtenue dans la transmission des contraintes de cisaillement à la couche adhésive 15 par le film 5, la couche adhésive 15 reste soumise à des efforts de cisaillements correspondant aux forces tangentielles appliquées par la bande de contention 2 sur le dispositif de retenue 1. Le risque est que ces forces tangentielles entraînent, non pas un décollement de la peau ou du film 5 de la couche adhésive 15, mais une rupture cohésive de la couche adhésive 15, c'est-à-dire qu'elle se sépare en deux parties collées l'une sur la peau et l'autre sur le film 5. Dans le cas d'une couche adhésive 15' de type double face, ce phénomène de rupture cohésive peut intervenir pour les deux couches adhésives élémentaires 16a, 16b.

Le déposant a donc aussi étudié l'influence de ces forces tangentielles et a défini un paramètre supplémentaire permettant d'améliorer le dispositif de retenue 1, à savoir une résistance t au cisaillement tangentiel. La résistance t au cisaillement tangentiel quantifie la capacité de la couche adhésive à résister à une rupture cohésive. Le déposant a, en effet, constaté que plusieurs caractéristiques de la couche adhésive 15 entrent en jeu dans sa capacité à résister à une rupture cohésive : l'épaisseur e de la couche adhésive 15, sa viscosité et sa surface.

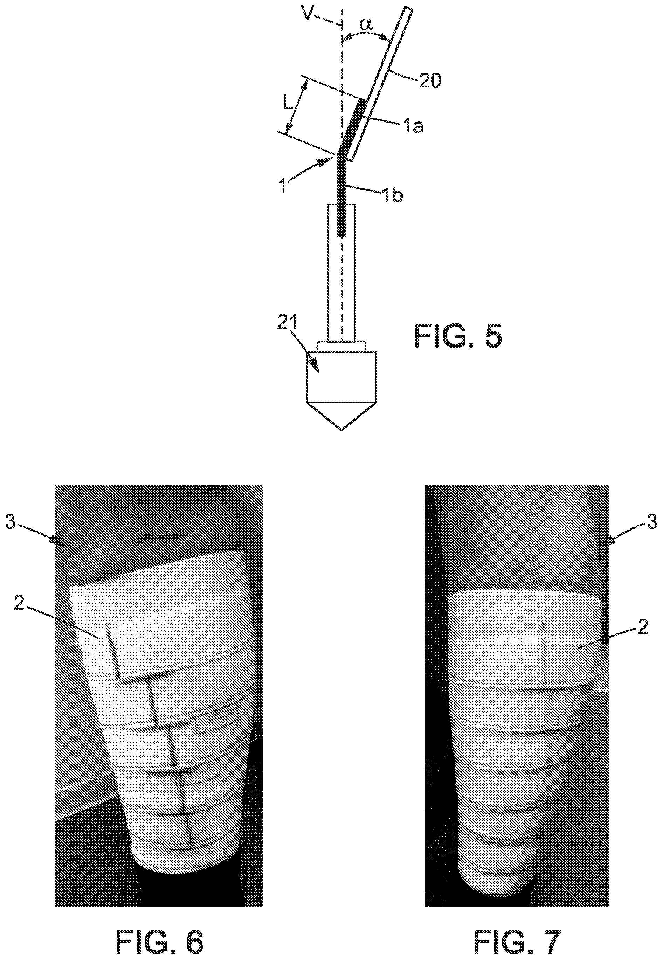

La résistance t au cisaillement tangentiel est mesurée par un test de fluage représenté sur la figure 5. Le principe de ce test de fluage consiste à déterminer le temps nécessaire pour obtenir une rupture cohésive de la couche adhésive soumise à l'action d'un poids déterminé qui simule les forces tangentielles appliquées au dispositif. Cette résistance t au cisaillement tangentiel est définie par le temps nécessaire pour constater la rupture cohésive.

Une éprouvette du dispositif de retenue 1 est découpée. Par exemple, l'éprouvette fait 10 cm de longueur et 2,5 cm de largeur. Une première partie de cette éprouvette est collée sur une plaque 20 de test étalon en acier en prenant bien soin de ne pas inclure de bulle d'air entre la couche adhésive 15 et la plaque 20 pour assurer un contact intime. La première partie 1a, collée, présente une longueur L, par exemple de 2,5 cm et une largeur I de 2,5 cm. Une pression sur la surface collée est appliquée afin d'éviter un décollement prématuré à cause d'un mauvais contact avec la plaque 20. Une deuxième partie 1b de l'éprouvette est laissée libre. Le test est réalisé à 23°C après conditionnement de 24 heures de l'ensemble plaque plus éprouvette à 23°C. La plaque 20 est installée sur un support de telle sorte qu'elle soit inclinée d'un angle α de 2° (degrés) par rapport à une direction verticale V, afin que les oscillations en début d'expérience après installation du lest ne viennent pas perturber les résultats. Un lest 21 de masse M est suspendu à la deuxième partie 1b de l'éprouvette qui pend depuis la plaque 20. Ceci déclenche automatiquement un compteur horaire. La rupture cohésive de la couche adhésive 15 qui entraine la chute du lest 21 stoppe le compteur horaire. Trois mesures sont réalisées et une valeur moyenne est calculée.

La résistance t au cisaillement tangentiel est alors définie par :

$$t = (L^2.l.\ \eta)\ /\ 2.e.M.g$$

où L est la longueur de la première partie 1a du dispositif de retenue 1 collée sur la plaque 20,

l est la largeur de la première partie 1a du dispositif de retenue 1,
$\eta$ est une viscosité de la couche adhésive 15,
e est une épaisseur de la couche adhésive 15,
M est la masse du lest 21 suspendu à la deuxième partie 1b du dispositif de retenue 1, M étant choisie en fonction de la contrainte tangentielle exercée par l'article de contention,

$$g = 9,81\ m/s^2$$

A titre d'exemple non limitatif, pour une bande de contention 2 à allongement court, le déposant a évalué que les contraintes tangentielles sont de l'ordre de 2 kPa à 5 kPa en fonction de la pression d'interface appliquée par la bande de contention 2, soit entre 30 mm et 80 mm de mercure. Sur cette base, il a déterminé qu'un lest 21 de 1 Kg est adapté aux contraintes de cisaillement impliquées par la bande de contention 2 à allongement court sur l'éprouvette précitée. Le rapport M/S pour une surface S de dispositif de retenue 1 comprise entre 20 cm$^2$ et 50 cm$^2$ (qui correspond à une fourchette préférée de surface) conduit à des contraintes comprises entre 2 kPa et 5 kPa et est donc bien représentatif des efforts appliqués par la bande de contention 2 à allongement court. Pour des raisons de facilité de mise en oeuvre du test une surface carrée de 6,25 cm$^2$ qui correspond à un rapport M/S de 16kPa a été choisie.

L'homme du métier pourra toutefois adapter la masse du lest 21 aux contraintes de cisaillement appliquées par l'article de contention.

Sur la base de ces éléments et après des tests aux limites, pour les substances adhésives qui présentent les plus faibles viscosités et les pouvoirs adhésifs les plus faibles, quel que soit le grammage d'enduction, il est possible de déterminer les limites de fluage pour la couche adhésive.

Il a été ainsi déterminé que la couche adhésive 15 soit, de préférence, choisie pour présenter une résistance t au cisaillement tangentiel d'au moins 10 heures pour que le dispositif de retenue 1 soit fonctionnel.

Le dispositif de retenue 1 selon l'invention peut être fabriqué selon un procédé de fabrication présentant les étapes suivantes. La couche adhésive 15 est réalisée sur un film provisoire siliconé et recouverte sur une face opposée au film provisoire d'un film protecteur temporaire et siliconé. Après retrait du film protecteur, la couche adhésive 15 est transférée sur la deuxième face 5b lisse du film 5. Des bobines de trois couches sont ainsi obtenues et découpées avec la forme choisie pour le dispositif de retenue 1. Le film provisoire siliconé sert de protecteur de la couche adhésive avant son retrait et collage sur la peau.

Le dispositif de retenue 1 peut se présenter sous toute forme appropriée, notamment géométrique, rectangle, rectangle avec coins arrondis, bi ou trilobé, banane ou autre. Ses dimensions sont choisies pour garantir le maintien de l'article de contention. Ses dimensions sont choisies pour que le dispositif de retenue 1 présente une surface comprise entre 10 cm$^2$ et 80 cm$^2$ en fonction du nombre et de la position des dispositifs de retenue 1 employés. De préférence, un seul dispositif de retenue 1 de forme rectangulaire, si nécessaire avec les angles très légèrement arrondis pour minimiser les risques de décollement, avec une surface comprise entre 20 cm$^2$ et 40 cm$^2$ sera utilisé pour retenir une bande de contention 2.

Dans le cas de forme géométrique complexe (par exemple une forme trilobée), la surface pourra être calculée de la façon suivante. Les feuilles de photocopieurs de papier A4 standard blanche présentent un grammage très précis, indiqué sur les emballages qui est de 80g/m$^2$. On découpe un échantillon de 100 cm$^2$ et on le pèse pour vérifier cette valeur. On pose le dispositif de retenue avec son protecteur provisoire sur une feuille A4 et dessine ses contours sur la feuille de papier 80 g/m$^2$. On découpe la forme obtenue et on la pèse. Connaissant le poids de 100 cm$^2$ on en déduit la surface exacte du dispositif.

Afin de faciliter le collage sur la peau et son maintien sur la jambe 3, le dispositif de retenue 1 peut présenter au moins un axe de symétrie.

Dans le cadre de la fixation d'une bande de contention 2, le dispositif de retenue 1 peut être positionné sous le genou horizontalement environ entre 3 cm et 8 cm selon la morphologie du patient. Il peut ainsi, par exemple, se présenter sous la forme d'une bande rectangulaire à bords arrondis qui entoure de façon incomplète (comme un bracelet) la jambe sous le genou au niveau de la dernière spire de la bande de contention 2. En variante, il peut être disposé verticalement sur la crête tibiale de manière à être recouvert par plusieurs spires de la bande de contention 2. Selon une autre variante, le « bracelet » peut être remplacé par plusieurs dispositifs de retenue 1 de dimensions inférieures à celles du « bracelet ». Il est également possible de combiner plusieurs des dispositifs de retenue précités.

Bien que décrit en relation avec une application à des bandes de contention 2 à allongement court, le dispositif de retenue peut être appliqué pour d'autres articles de contention pour éviter le glissement sur un membre inférieur voire toute autre partie du corps où un glissement serait susceptible d'intervenir suite aux mouvements. Bien entendu leur positionnement est alors adapté en conséquence.

**Exemples**

L'invention sera illustrée par les exemples et les tests vivo suivants.

**FABRICATIONS DU DISPOSITIF DE RETENUE 1**

**Exemple 1**

Préparation de la couche adhésive 15

On a préparé une couche adhésive 15 par mélange dans un malaxeur MEL G-40.
Sa composition, exprimée en pourcentage en poids par rapport au poids total de la composition est la suivante :

- Huile minérale commercialisée par la société Shell sous la dénomination Ondina ®919 : 39,7%,
- Sel de sodium de carboxyméthylcellulose (hydrocolloïde) commercialisé par la société AQUALON sous la dénomination CMC Blanose ®7H4XF : 14,8%,
- Copolymère séquencé de poly(styrène-éthylène-butylène-styrène) (élastomère) commercialisé par la société KRATON sous la dénomination KRATON® G 1651 E : 4,7%,
- Antioxydant commercialisé sous la dénomination IRGANOX® 1010 par la société CIBA SPECIALTY CHEMICALS : 0,2%,
- Résine tackifiante commercialisée par la société EXXON CHEMICALS sous la dénomination ESCOREZ® 5380 : 35,6%,
- Copolymère de sel de l'acide 2-méthyl-2[(1-oxo-2-propényl)amino]-1-propanesulfonique et de l'ester 2-hydroxyéthyle de l'acide propénoïque commercialisé par la société SEPPIC sous la dénomination SEPINOV® EMT 10 : 5%.

On a introduit les différents constituants à une température comprise entre 105 et 115°C sous agitation, de manière à obtenir un mélange homogène.
Plus précisément, on a introduit initialement l'huile minérale, l'hydrocolloïde, et l'élastomère puis l'antioxydant, le SEPINOV® EMT 10 et enfin la résine tackifiante.

Préparation du dispositif de retenue 1

On enduit à un grammage de 100 g/m$^2$ sur un film provisoire siliconé cet adhésif et on recouvre la couche adhésive 15 avec un film protecteur temporaire siliconé.
On transfère cette couche adhésive 15 sur un film 5 pourvu de perforations de la société Tredegar dont la référence est X33929 qui présente une hauteur de lèvres h de 0,204 mm et une somme des longueurs des bords libres 8 de 19 cm/cm$^2$.
On découpe le dispositif de retenue 1 final qui se présente sous une forme rectangulaire de 12 cm de longueur et 2,8 cm de largeur soit une surface de 33,6 cm$^2$.
On a évalué la résistance t au cisaillement tangentiel de ce dispositif de retenue 1 selon le test de fluage décrit précédemment. On obtient une valeur de 21,3 heures. A cette valeur, on constate la rupture cohésive de la couche adhésive 15. Ce dispositif de retenue 1 remplit donc la caractéristique de résistance au cisaillement tangentiel supérieure à 10 heures.

**Exemple 2**

Préparation de la couche adhésive 15

On a préparé une couche adhésive 15 par mélange dans un malaxeur MEL G-40.
Sa composition, exprimée en pourcentage en poids par rapport au poids total de la composition est la suivante :

- Huile minérale commercialisée par la société Shell Chemicals sous la dénomination Edelex 945 : 9,76 %,

- Copolymère séquencé de poly (styrène-isoprène-styrène) (élastomère) commercialisé par la société KRATON sous la dénomination KRATON® D 1111KT : 13,07 %,
- Copolymère séquencé de poly (styrène-isoprène-styrène) (élastomère) commercialisé par la société Exxon Mobil Chemical sous la dénomination VECTOR 4114A : 6,44 %,
- Lanoline : 7,81 %
- Antioxydant commercialisé sous la dénomination IRGANOXO 565 par la société CIBA SPECIALTY CHEMICALS : 0,1 %,
- Résine tackifiante commercialisée par la société Parchem sous la dénomination Dertoline P2L : 39,03 %,
- Perkacit ZDBC antioxydant commercialisé par la société AKZO : 0,39 %,
- Oxyde de zinc : 21,86 %,
- Dioxyde de titane commercialisé par la société Cristal sous la dénomination TIONA 595 : 1,54 %.

On a introduit les différents constituants à une température comprise entre 130°C et 140°C sous faible agitation, de manière à obtenir un mélange homogène.

Plus précisément, on a introduit initialement l'huile minérale, les deux élastomères, les deux antioxydants et le dioxyde de titane, puis l'oxyde de zinc ensuite la résine tackifiante et pour finir la lanoline.

Préparation du dispositif de retenue 1

On enduit à un grammage de 80 g/m$^2$ sur un film provisoire siliconé cet adhésif et on recouvre la couche adhésive 15 avec un film protecteur temporaire siliconé.

On transfère cette couche adhésive 15 sur un film 5 blanc pourvus de performations de la société RKW dont la référence est 45107 qui présente une hauteur de lèvres h de 0,445 mm et une somme des longueurs des bords libres de 18,3 cm/cm$^2$.

On découpe le dispositif de retenue 1 final qui se présente sous la même forme que celle de l'exemple 1.

On a évalué la résistance t au cisaillement tangentiel de ce dispositif de retenue 1 selon le test de fluage décrit précédemment. Après 165 heures, aucune rupture cohésive de la couche adhésive 15 n'est constatée. Ce dispositif de retenue 1 remplit donc la caractéristique de résistance au cisaillement tangentiel supérieure à 10 heures.

**Exemple 3**

On utilise une couche adhésive 15' de type double face à usage médical commercialisée par la société ADHEX sous la référence P754.

On applique par pression la face « vue » du double face sur la deuxième face 5b lisse d'un film 5 pourvu de perforations de la société Tredegar dont la référence est référence X34434 et qui présente une hauteur de lèvres h de 0,455 mm et une somme des longueurs des bords libres 8 de 18,4 cm/cm$^2$.

On découpe le dispositif de retenue 1 final qui se présente sous une forme rectangulaire de 14 cm de longueur et 2,8 cm de largeur soit une surface de 39,2 cm$^2$.

On a évalué la résistance t au cisaillement tangentiel de ce dispositif de retenue 1 selon le test de fluage décrit précédemment. Après 140 heures, aucune rupture cohésive d'aucune des deux couches adhésives élémentaires 16a, 16b n'est constatée. Ce dispositif de retenue 1 remplit donc la caractéristique de résistance au cisaillement tangentiel supérieure à 10 heures.

**TEST IN VIVO**

Sur les figures 6 et 7, on a évalué sur le test in vivo décrit ci-après le glissement et le décalage spire sur spire au cours du temps de diverses bandes de contention 2.

Le mode opératoire de ce test in vivo est le suivant.

On enroule la ou les bandes de contention 2 autour de la jambe 3 selon les préconisations décrites dans la notice du système bicouches K2.

Pour rappel, cette notice préconise la méthode d'application suivante :

1) tenir le pied à 90°, orteils vers le haut ; appliquer KTECH à la base des orteils en faisant deux tours d'ancrage, en s'assurant que la face ouatée soit en contact direct avec la peau et que l'indicateur de pression soit situé sur le côté supérieur de la bande ; continuer en faisant une figure en « 8 » autour de la cheville, sans appliquer une tension excessive sur le pied et en recouvrant bien le talon ;

2) remonter jusqu'au genou en effectuant des spirales et en étirant la bande de manière appropriée : l'indicateur de pression imprimé sur les bandes doit former un cercle ; pour obtenir un recouvrement correct, l'indicateur de pression doit être tout juste recouvert (recouvrement de 50%) ; finir 2cm sous le genou et couper l'excédent de bande ; 3) appliquer KPRESS sur KTECH selon la même technique en commençant un doigt au-dessus de KTECH et en finissant un doigt sous KTECH afin que seule KTECH soit en contact direct avec la peau ; une fois appliquée, appuyer doucement sur le bandage avec les mains pour assurer une bonne tenue du système.

On comprend bien que cette dernière étape 3) n'est pas nécessaire si on n'utilise pas une seconde bande de contention auto adhérente. Dans ce cas, la dernière spire est fixée sur elle-même à l'aide d'une attache métallique, d'un sparadrap ou, de préférence, avec la partie à crochets d'un Velcro®, marque déposée. Toutefois, on appuie à nouveau doucement sur le bandage avec les mains pour assurer une bonne tenue du système de contention.

On trace à l'aide d'un feutre fin et non effaçable un trait vertical sur au moins trois spires, sur l'axe de la crête tibiale, à partir de la dernière spire enroulée. Cette marque sert de référence pour évaluer à l'aide d'un réglet gradué au mm, le décalage horizontal du trait au terme de la durée du test. Lors des mouvements, ce trait perd de son caractère rectiligne et se présente en échelons d'autant plus décalés que les glissements spires sur spires sont importants. Si le glissement spire sur spire est très faible ou inexistant, le trait vertical reste intègre ou varie très peu principalement sur la première spire qui se situe sous la dernière spire enroulée.

Ce décalage du trait vertical est représentatif du relâchement de la bande et illustre son glissement potentiel au cours du temps. Le test dure 6 heures. On mesure au bout des 6 heures le décalage du trait vertical sur les 3 premières spires.

On trace aussi à l'aide du feutre fin et non effaçable un trait horizontal au-dessus de la dernière spire enroulée. Cette marque sert de référence pour évaluer à l'aide d'un réglet gradué au mm, le décalage vertical entre ce trait et la position sur la jambe de la dernière spire au terme de la durée du test. On considère que si l'écart entre le trait horizontal et la dernière spire est, au bout de 6 heures, supérieur à 15 mm la bande glissera complètement avant 24 heures.

Si l'on utilise une bande avec le dispositif de retenue selon l'invention, on retire le protecteur provisoire de la couche adhésive et on colle le dispositif de retenue soit horizontalement sous le genou de manière à être recouvert par la dernière spire, soit verticalement sur la crête tibiale de manière à être recouvert par plusieurs spires. On enroule ensuite la bande comme précédemment sur la jambe. Ainsi la ou les dernières spires recouvrent le dispositif de retenue.

On a réalisé ce test vivo avec les bandes suivantes.

Le système de contention bicouches commercialisé par la société Laboratoires URGO sous la dénomination K2 taille 18 cm-25 cm qui est constitué de deux bandes de contention. Une première bande Ktech qui est un allongement court et applique la majorité de la pression et une seconde bande Kpress auto adhérente qui applique le résiduel de pression mais surtout fixe la première bande et évite son glissement et le décalage spire sur spire. On a aussi testé la bande Ktech seule sans et avec divers dispositifs de retenue de l'invention. Cette bande Ktech est étalonnée et elle est posée selon le pictogramme d'étalonnage.

Un tricot 3D, qui est un allongement court, commercialisé en Allemagne par la société Neumed. Ce tricot à base de fibres synthétiques présente une face alvéolée qui vient en contact avec la peau et une face pleine opposée. La contrainte de cisaillement de ce tricot mesurée comme décrit dans le document WO 2017/089731 est de 2020 Pa. Cette bande de contention présente donc un fort décalage spire sur spire qui conduit à son glissement. La bande de contention employée est une bande de 5 m de longueur, de 8 cm de largeur et présente un grammage de 219 g/m² et une épaisseur de 1,44 mm.

Pour tester cette bande de contention Neumed, on a déterminé son allongement à la pose en fonction de la pression de travail recherchée, par exemple à l'aide de la courbe traction rupture telle que définie dans la norme EN ISO 13934-1. Le principe de cette norme est le suivant.

On découpe ainsi une bande rectangulaire de largeur suffisante en l'effilochant si besoin pour obtenir un échantillon de largeur finale de 50 mm. On place cet échantillon dans les mors d'un dynamomètre distants de 200 mm. On procède à l'essai de traction jusqu'à la rupture de l'échantillon à la vitesse de 100 mm/mn. On répète ainsi le test pour cinq échantillons. Les conditions de conditionnement, d'hygrométrie et de température sont définies dans la norme EN ISO 13934 - 1.

D'après la loi de Laplace, l'allongement à effectuer correspond à la pression recherchée.

On a ainsi déterminé un allongement à la pose de 45 % pour la bande de contention Neumed de manière à appliquer à la pose une pression de travail maximale de l'ordre de 60 mm à 70 mm de mercure.

Pour poser la bande de façon appropriée, on a étalonné cette bande à 45 % à l'aide d'un pochoir comme décrit dans le document WO 2007/113340, page 13, ligne 18 à page 14, ligne 6.

Selon un autre exemple, la bande utilisée est une bande de 5 mètres de longueur et 10 cm de largeur constituée d'un tissé, qui est un allongement court, commercialisé par la société Lohmann Rauscher sous la dénomination Rosidal K®. Elle a été posée comme les bandes précédentes avec un recouvrement de 50%. La dernière spire est fixée sur elle-même à l'aide des 2 attaches métalliques présentes dans la boite.

Cette bande ne possède pas d'étalonnage. Pour obtenir comme pour les bandes précédentes une pression de travail de l'ordre de 60 à 70 mm de mercure, on a posé cette bande en extension de manière à obtenir une pression de repos à la pose de l'ordre de 40 mm de mercure. Dans ce but, on a disposé avant la pose de la bande un capteur de pression commercialisé par la société Microlab Ellectronica sous la dénomination PicoPress® au point B1 sur la jambe. Ce point, couramment utilisé pour mesurer les pressions appliquées par une bande, correspond à la zone où le tendon d'Achille se transforme en muscle du mollet, soit de manière générale 10 à 15 cm environ au-dessus de la mallèole.

On a appliqué sur la jambe gauche la bande Rosidal K® seule et sur la jambe droite le dispositif de retenue de l'exemple 3 sur la crête tibiale recouvert par la bande Rosidal K®.

Les résultats obtenus lors de ces différents tests sont rassemblées dans le tableau 1.

[Tableau 1]

| Bande avec ou sans dispositif | Allongement à la pose | Décalage vertical En mm | Spire1 Décalage En mm | Spire2 Décalage En mm | Spire 3 Décalage En mm | Position du dispositif |
|---|---|---|---|---|---|---|
| Ktech seule | Selon étalonnage | 29 | 10 | 8 | 2 | Sans |
| Ktech + exemple 3 | Selon étalonnage | 0 | 12 | 0 | 0 | Bracelet sous genou |
| Ktech + exemple 2 | Selon étalonnage | 2 | 0 | 0 | 0 | Crête tibiale |
| Neumed seule | 45 % | 20 | 25 | 11 | 5 | Sans |
| Neumed + exemple 1 | 45 % | 0 | 0 | 0 | 0 | Crête tibiale |
| K2 Ktech +Kpress | Selon étalonnage | 0 | 0 | 0 | 0 | Sans |
| Rosidal K® seule | Pression de repos au point B1 42 mm de mercure | 49 | 8 | 4 | 2 | Sans |
| Rosidal K®+ exemple 3 | Pression de repos au point B1 42 mm de mercure | 0 | 0 | 0 | 0 | Crête tibiale |

Ce tableau illustre les avantages des dispositifs selon l'invention.

On observe pour le système K2 aucun décalage vertical et aucun décalage spire sur spire. Un tel résultat est cohérent, du fait de la « cohésivation » de la seconde bande auto adhérente qui bloque le glissement des spires les unes sur les autres et le glissement vertical. En l'absence de cette dernière la première bande Ktech présente un décalage vertical et spire sur spire important. Elle ne peut tenir seule et glissera rapidement. Son usage avec le dispositif de retenue de l'exemple 2 disposé sur la crête tibiale supprime le décalage spire sur spire et élimine le glissement de la bande de contention. La valeur de 2 mm de décalage vertical est négligeable. On considère qu'une valeur moyenne sur plusieurs personnes de l'ordre de 4 mm à 5 mm n'est pas représentative du potentiel de glissement d'une bande de contention au cours du temps et représente les aléas de mesures liés à la variation des mollets des testeurs, la reproductibilité de la pose et les variabilités de fabrication des bandes. De plus, on considère qu'un décalage de 1 mm représente l'incertitude sur l'évaluation de la mesure de l'épaisseur du trait, et est donc négligeable.

On retrouve un résultat similaire avec le dispositif de retenue de l'exemple 3 dans lequel ce dernier se présente sous la forme d'une bande longitudinale mais disposée cette fois sous le genou en « bracelet », c'est-à-dire en entourant la face et les côtés de la bande sous la dernière et l'avant dernière spire. On bloque faiblement (valeur de 12 mm) la spire 1. Mais en l'absence de décalage spire sur spire sur les spires 2 et 3 suivantes on diminue significativement le risque de glissement à terme. Toutefois, cela indique qu'il est dans ce cas préférable d'utiliser le dispositif anti glissement sur la crête tibiale. Ceci est confirmé par les résultats de la bande de contention Neumed avec le dispositif de retenue de l'exemple 1 qui, sur la crête tibiale, bloque tout glissement vertical ou spire sur spire. De même, on obtient un résultat identique avec le dispositif de retenue de l'exemple 3 disposé sur la crête tibiale avec cette fois une bande, qui est un tissé, la bande Rosidal K®.

De façon générale, ces résultats montrent que les dispositifs de retenue selon l'invention permettent d'éviter le glissement des bandes de contention à allongement court et d'utiliser une seule bande de ce type à la place d'un système bicouche comprenant une bande auto-adhérente avec latex. Bien évidemment de tels dispositifs de retenue pourront aussi s'appliquer à d'autres types de bandes ou articles de contention et leur position adaptée pour éviter le glissement desdits articles.

**Revendications**

1. Dispositif de retenue (1, 1') destiné à prévenir un glissement d'un article de contention sur un membre d'un individu, le dispositif de retenue (1, 1') comprenant :

    - un film (5) souple et inextensible présentant une première face (5a) destinée à être placée en contact avec l'article de contention, et une deuxième face (5b) opposée à la première face et destinée à être placée en regard du membre de l'individu, la première face (5a) comportant une surface de base (6) et une pluralité de saillies (7) s'étendant chacune depuis la surface de base (6) jusqu'à un bord libre (8), chaque saillie (7) présentant une hauteur (h) entre la surface de base (6) et le bord libre (8) comprise entre 80 $\mu$m et 1200 $\mu$m, de préférence entre 150 $\mu$m et 900 $\mu$m, et étant conformée en une paroi (10) dont le bord libre (8) a une longueur, les saillies (7) étant agencées sur la surface de base (6) de telle manière qu'une somme des longueurs des bords libres (8) par unité de surface de la surface de base (6) soit comprise entre 5 cm/cm$^2$ et 120 cm/cm$^2$, de préférence entre 10 cm/cm$^2$ et 80 cm/cm$^2$,
    - une couche adhésive (15, 15') recouvrant au moins en partie la deuxième face (5b) du film (5) et adaptée pour retenir le film (5) sur le membre de l'individu.

2. Dispositif de retenue (1, 1') selon la revendication 1, dans lequel la paroi (10) de chaque saillie (7) s'étend autour d'un axe central (A) sensiblement normal à la surface de base (6), la longueur du bord libre (8) de chaque saillie (7) définissant un périmètre.

3. Dispositif de retenue (1, 1') selon l'une quelconque des revendications 1 et 2, dans lequel les saillies (7) sont formées en une seule pièce avec la surface de base (6).

4. Dispositif de retenue (1, 1') selon l'une quelconque des revendications 1 à 3, dans lequel le film (5) comporte une pluralité de perforations (9) réalisées au travers du film (5) depuis la deuxième face (5b) de manière à obtenir des déformations locales du film (5) sur la première face (5a), chaque déformation locale entourant l'une des perforations (9) et formant la paroi (10) de l'une des saillies (7).

5. Dispositif de retenue (1, 1') selon l'une quelconque des revendications 1 à 4, dans lequel les saillies (7) sont agencées selon un réseau ayant une densité comprise entre 15 saillies/cm$^2$ et 700 saillies/cm$^2$, de préférence entre 80 saillies/cm$^2$ et 300 saillies/cm$^2$.

6. Dispositif de retenue (1, 1') selon l'une quelconque des revendications 1 à 5, dans lequel la deuxième face (5b) du film (5) présente une surface lisse.

7. Dispositif de retenue (1, 1') selon l'une quelconque des revendications 1 à 6, dans lequel le film (5) est réalisé en polymère thermoplastique, notamment en polymère à base de polyoléfines, tels que du polyéthylène, en copolymère de polyéthylène et d'acétate de vinyle, en copolymère de polyéthylène et d'acrylate de butyle ou de polychlorure de vinyle plastifié, en polymère ou copolymère de polyester, en polymère ou copolymère de polyamide, en polymère ou copolymère de polyuréthanne ou en polymère de polycarbonate.

8. Dispositif de retenue (1, 1') selon la revendication 7, dans lequel le film (5) a un grammage compris entre 10 g/m$^2$ et 120 g/m$^2$, de préférence entre 20 g/m$^2$ et 60 g/m$^2$.

9. Dispositif de retenue (1, 1') selon l'une quelconque des revendications 1 à 8, dans lequel la couche adhésive (15, 15') présente une résistance t au cisaillement tangentiel d'au moins 10 heures selon un test de fluage dans lequel une première partie (1a) du dispositif de retenue (1) est collée par l'intermédiaire de la couche adhésive (15, 15') à une plaque (20) inclinée d'un angle de 2°, par rapport à une direction verticale (V), en laissant libre une deuxième partie (1b) du dispositif de retenue (1), un lest (21) étant suspendu à la deuxième partie (1b) du dispositif de retenue (1), avec : t = (L$^2$.l. $\eta$) / 2.e.M.g où L est une longueur de la couche adhésive (15, 15') de la première partie (1a) du dispositif de retenue (1, 1'),

    l est une largeur de la couche adhésive (15, 15') de la première partie (1a) du dispositif de retenue (1, 1'),

η est une viscosité de la couche adhésive (15, 15'),

e est une épaisseur de la couche adhésive (15, 15'),

M est une masse du lest (21), M étant choisie en fonction d'une contrainte tangentielle exercée par l'article de contention,

g = 9,81 m/s$^2$.

10. Dispositif de retenue (1, 1') selon l'une quelconque des revendications 1 à 8, dans lequel la couche adhésive (15, 15') est constituée d'une substance adhésive sensible à la pression et notamment d'une substance adhésive choisie parmi :

- les adhésifs acryliques à base de polymères acrylates ou de copolymères d'acide acrylique et d'esters de l'acide acrylique,
- les adhésifs à base de poly-isobutylène de haut poids moléculaires,
- les adhésifs à base de poly-isoprène naturel ou synthétique,
- les adhésifs à base de polymères ou copolymères de siloxane et de résines tackifiantes ou les gels de silicone à base de polyxilosanes réticulés,
- les adhésifs à base de polyuréthanne ou les gels de polyuréthanne,
- les adhésifs à base de copolymères triblocs, de plastifiants et de résines tackifiantes, en particulier les polymères triblocs du type styrène-séquence centrale-styrène dans lequel la séquence centrale est insaturée, par exemple isoprène ou butadiène, ou saturée, par exemple éthylène-butylène ou éthylène-propylène, éventuellement mélangés avec des copolymères diblocs comme par exemple des copolymères styrène-isoprène ou styrène-butadiène.

11. Dispositif de retenue (1) selon la revendication 10, dans lequel la couche adhésive (15) comporte une unique couche adhésive élémentaire (16), la couche adhésive élémentaire (16) ayant notamment un grammage d'au moins 50 g/m$^2$, de préférence compris entre 60 g/m$^2$ et 120 g/m$^2$.

12. Dispositif de retenue (1) selon la revendication 11, dans lequel la couche adhésive élémentaire (16) a une force d'adhérence comprise entre 0,5 N/cm et 5 N/cm mesurée par un test de pelage dans lequel la force d'adhérence selon un angle de 90° pour décoller la couche adhésive (15) d'une surface de référence choisie parmi la deuxième face (5b) du film (5) et une surface de peau est mesurée.

13. Dispositif de retenue (1') selon la revendication 10, dans lequel la couche adhésive (15') comprend :

- une première couche adhésive élémentaire (16a) destinée à être mise en contact avec le membre, la première couche adhésive élémentaire (16a) ayant notamment un grammage compris entre 25 g/m$^2$ et 120 g/m$^2$, de préférence entre 40 g/m$^2$ et 80 g/m$^2$,
- une âme (17) présentant une première surface portant la première couche adhésive élémentaire (16a), et une deuxième surface opposée à la première surface,
- une deuxième couche adhésive élémentaire (16b) pour fixer la deuxième surface de l'âme (17) à la deuxième face (5b) du film (5), la deuxième couche adhésive élémentaire (16b) ayant notamment un grammage compris entre 20 g/m$^2$ et 120 g/m$^2$, de préférence entre 30 g/m$^2$ et 50 g/m$^2$.

14. Dispositif de retenue (1') selon la revendication 13, dans lequel la première couche adhésive élémentaire (16a) a une force d'adhérence comprise entre 0,5 N/cm et 5 N/cm et la deuxième couche adhésive élémentaire (16b) a une force d'adhérence comprise entre 0,5 N/cm et 10 N/cm, la force d'adhérence de chacune des couches adhésives élémentaires choisie parmi les première (16a) et deuxième (16b) couches adhésives élémentaires étant mesurée par un test de pelage dans lequel la force d'adhérence selon un angle de 90° pour décoller ladite couche adhésive élémentaire d'une surface de référence choisie parmi la deuxième face (5b) du film (5) et une surface de peau est mesurée.

15. Système de contention comprenant un article de contention destiné à être placé sur un membre d'un patient, tel qu'une bande de contention (2) textile, notamment à allongement court, destinée à être enroulée sur le membre, et un dispositif de retenue (1) selon l'une quelconque des revendications 1 à 14.

**Patentansprüche**

1. Haltevorrichtung (1, 1'), welche dazu vorgesehen ist, ein Abgleiten eines Stützartikels an einem Körperglied einer Person zu verhindern, wobei die Haltevorrichtung (1, 1') umfasst:

   - einen flexiblen und nicht dehnbaren Film (5), welcher eine erste Seite (5a), welche dazu vorgesehen ist, in Kontakt mit dem Stützartikel platziert zu sein, und eine zweite Seite (5b) gegenüber der ersten Seite aufweist, welche dazu vorgesehen ist, gegenüber dem Körperglied der Person platziert zu sein, wobei die erste Seite (5a) eine Basisfläche (6) und eine Mehrzahl von Vorsprüngen (7) umfasst, welche sich jeweils von der Grundfläche (6) bis zu einem freien Rand (8) erstrecken, wobei jeder Vorsprung (7) eine Höhe (h) zwischen der Basisfläche (6) und dem freien Rand (8) aufweist, welche zwischen 80 $\mu$m und 1200 $\mu$m beträgt, vorzugsweise zwischen 150 $\mu$m und 900 $\mu$m, und zu einer Wand (10) geformt ist, deren freier Rand (8) eine Länge aufweist, wobei die Vorsprünge (7) derart an der Basisfläche (6) angeordnet sind, dass eine Summe der Längen der freien Ränder (8) pro Einheitsfläche der Basisfläche (6) zwischen 5 cm/cm$^2$ und 120 cm/cm$^2$ beträgt, vorzugsweise zwischen 10 cm/cm$^2$ und 80 cm/cm$^2$,
   - eine Haftschicht (15, 15'), welche wenigstens teilweise die zweite Seite (5b) des Films (5) bedeckt und dazu eingerichtet ist, den Film (5) an dem Körperglied der Person zu halten.

2. Haltevorrichtung (1, 1') nach Anspruch 1, wobei sich die Wand (10) von jedem Vorsprung (7) um eine Mittelachse (A) erstreckt, welche im Wesentlichen senkrecht zu der Basisfläche (6) ist, wobei die Länge des freien Rands (8) von jedem Vorsprung (7) einen Umfang definiert.

3. Haltevorrichtung (1, 1') nach einem der Ansprüche 1 und 2, wobei die Vorsprünge (7) einstückig mit der Basisfläche (6) gebildet sind.

4. Haltevorrichtung (1, 1') nach einem der Ansprüche 1 bis 3, wobei der Film (5) eine Mehrzahl von Perforationen (9) umfasst, welche durch den Film (5) hindurch von der zweiten Seite (5b) aus derart gebildet sind, dass lokale Verformungen des Films (5) an der ersten Seite (5a) erhalten werden, wobei jede lokale Verformung eine der Perforationen (9) umgibt und die Wand (10) von einem der Vorsprünge (7) bildet.

5. Haltevorrichtung (1, 1') nach einem der Ansprüche 1 bis 4, wobei die Vorsprünge (7) gemäß einem Netz angeordnet sind, welches eine Dichte aufweist, welche zwischen 15 Vorsprüngen/cm$^2$ und 700 Vorsprüngen/cm$^2$ beträgt, vorzugsweise zwischen 80 Vorsprüngen/cm$^2$ und 300 Vorsprüngen/cm$^2$.

6. Haltevorrichtung (1, 1') nach einem der Ansprüche 1 bis 5, wobei die zweite Seite (5b) des Films (5) eine glatte Oberfläche aufweist.

7. Haltevorrichtung (1, 1') nach einem der Ansprüche 1 bis 6, wobei der Film (5) aus thermoplastischem Polymer, insbesondere aus Polymer auf Basis von Polyolefinen, wie beispielsweise Polyethylen, aus Co-Polymer von Polyethylen und Vinylacetat, aus Co-Polymer von Polyethylen und Butylacrylat oder plastifiziertem Polyvinylchlorid, aus Polymer oder Co-Polymer von Polyester, aus Polymer oder Co-Polymer von Polyamid, aus Polymer oder Co-Polymer von Polyurethan oder aus Polymer von Polycarbonat gebildet ist.

8. Haltevorrichtung (1, 1') nach Anspruch 7, wobei der Film (5) ein Flächengewicht aufweist, welches zwischen 10 g/m$^2$ und 120 g/m$^2$ beträgt, vorzugsweise zwischen 20 g/m$^2$ und 60 g/m$^2$.

9. Haltevorrichtung (1, 1') nach einem der Ansprüche 1 bis 8, wobei die Haftschicht (15, 15') einen Widerstand t gegen tangentiales Scheren von wenigstens 10 Stunden gemäß einem Kriechtest aufweist, in welchem ein erster Teil (1a) der Haltevorrichtung (1) mittels der Haftschicht (15, 15') auf eine Platte (20) geklebt ist, welche um einen Winkel von 2° gegenüber einer vertikalen Richtung (V) geneigt ist, wobei ein zweiter Teil (1b) der Haltevorrichtung (1) frei belassen wird, wobei eine Last (21) an dem zweiten Teil (1b) der Haltevorrichtung (1) aufgehängt ist, mit: t = ($L^2$.l.$\eta$)/2.e.M.g, wobei L eine Länge der Haftschicht (15, 15') des ersten Teils (1a) der Haltevorrichtung (1, 1') ist,

   l eine Breite der Haftschicht (15, 15') des ersten Teils (1a) der Haltevorrichtung (1, 1') ist,
   $\eta$ eine Viskosität der Haftschicht (15, 15') ist,
   e eine Dicke der Haftschicht (15, 15') ist,
   M eine Masse der Last (21) ist, wobei M als Funktion einer lateralen Beschränkung ausgewählt ist, welche durch den Stützartikel ausgeübt wird,

$g = 9,81 \text{ m/s}^2$.

10. Haltevorrichtung (1, 1') nach einem der Ansprüche 1 bis 8, wobei die Haftschicht (15, 15') aus einer Haftsubstanz gebildet ist, welche druckempfindlich ist, und insbesondere aus einer Haftsubstanz, ausgewählt aus:

- Acryl-Haftmitteln auf Basis von Acryl-Polymeren oder Co-Polymeren von Acrylsäure und Acrylsäure-Estern,
- Haftmitteln auf Basis von Poly-Isobutan mit hohem Molekulargewicht,
- Haftmitteln auf Basis von natürlichem oder synthetischem Poly-Isopren,
- Haftmitteln auf Basis von Polymeren oder Co-Polymeren von Siloxan und haftungsfördernden Harzen oder Silikon-Gelen auf Basis von vernetzten Polyxilosanen,
- Haftmitteln auf Basis von Polyurethan oder Polyurtethan-Gelen,
- Haftmitteln auf Basis von Triblock-Copolymeren, Weichmachern und haftungsfördernden Harzen, insbesondere Triblock-Polymeren vom Styren-Zentralsequenz-Styren-Typ, in welchen die Zentralsequenz ungesättigt ist, beispielsweise Isopren oder Butadien, oder gesättigt, beispielsweise Ethylen-Butylen oder Ethylen-Propylen, ggf. gemischt mit Diblock-Co-Polymeren, wie beispielsweise Styren-Isopren- oder Styren-Butadien-Co-Polymeren.

11. Haltevorrichtung (1) nach Anspruch 10, wobei die Haftschicht (15) eine einzelne elementare Haftschicht (16) umfasst, wobei die elementare Haftschicht (16) insbesondere ein Flächengewicht von wenigstens 50 g/m$^2$ aufweist, vorzugsweise von zwischen 60 g/m$^2$ und 120 g/m$^2$.

12. Haltevorrichtung (1) nach Anspruch 11, wobei die elementare Haftschicht (16) eine Haftkraft aufweist, welche zwischen 0,5 N/cm und 5 N/cm beträgt, gemessen durch einen Ablösetest, in welchem die Haftkraft gemäß einem Winkel von 90° für ein Abziehen der Haftschicht (15) von einer Referenzfläche gemessen wird, welche aus der ersten Seite (5b) des Films (5) und einer Hautfläche ausgewählt wird.

13. Haltevorrichtung (1') nach Anspruch 10, wobei die Haftschicht (15') umfasst:

- eine erste elementare Haftschicht (16a), welche dazu vorgesehen ist, in Kontakt mit dem Körperglied versetzt zu sein, wobei die erste elementare Haftschicht (16a) insbesondere ein Flächengewicht von zwischen 25 g/m$^2$ und 120 g/m$^2$ aufweist, vorzugsweise von zwischen 40 g/m$^2$ und 80 g/m$^2$,
- einen Kern (17), welcher eine erste Oberfläche aufweist, welche die erste elementare Haftschicht (16a) trägt, sowie eine zweite Oberfläche gegenüber der ersten Oberfläche,
- eine zweite elementare Haftschicht (16b) für ein Fixieren der zweiten Oberfläche des Kerns (17) an der zweiten Seite (5b) des Films (5), wobei die zweite elementare Haftschicht (16b) insbesondere ein Flächengewicht aufweist, welches zwischen 20 g/m$^2$ und 120 g/m$^2$ beträgt, vorzugsweise zwischen 30 g/m$^2$ und 50 g/m2.

14. Haltevorrichtung (1') nach Anspruch 13, wobei die erste elementare Haftschicht (16a) eine Haftkraft aufweist, welche zwischen 0,5 N/cm und 5 N/cm beträgt, und die zweite elementare Haftschicht (16b) eine Haftkraft aufweist, welche zwischen 0,5 N/cm und 10 N/cm beträgt, wobei die Haftkraft von jeder der elementaren Haftschichten, ausgewählt aus der ersten (16a) und zweiten (16b) elementaren Haftschicht durch einen Ablösetest gemessen wird, in welchem die Haftkraft gemäß einem Winkel von 90° für ein Abziehen der elementaren Haftschicht von einer Referenzfläche gemessen wird, welche ausgewählt wird aus der zweiten Seite (5b) des Films (5) und einer Hautfläche.

15. Stützsystem, umfassend einen Stützartikel, welcher dazu vorgesehen ist, an einem Körperglied eines Patienten platziert zu sein, wie beispielsweise ein textiles Stützband (2), insbesondere mit niedriger Dehnung, welches dazu vorgesehen ist, an das Körperglied gewickelt zu sein, sowie eine Haltevorrichtung (1) nach einem der Ansprüche 1 bis 14.

## Claims

1. A holding device (1, 1') intended to prevent slippage of a retention garment on a limb of an individual, the holding device (1, 1') comprising:

- a flexible and inextensible film (5) having a first face (5a) intended to be placed in contact with the retention garment, and a second face (5b) opposite to the first face and intended to be placed opposite the limb of the individual, the first face (5a) including a base surface (6) and a plurality of protrusions (7) each extending from the base surface (6) up to a free edge (8), each protrusion (7) having a height (h) between the base surface (6) and the free edge (8) comprised between 80 $\mu$m and 1,200 $\mu$m, preferably between 150 $\mu$m and 900 $\mu$m, and being shaped as a wall (10) the free edge (8) of which has a length, the protrusions (7) being arranged over the base surface (6) such that a sum of the lengths of the free edges (8) per unit area of the base surface (6) is comprised between 5 cm/cm$^2$ and 120 cm/cm$^2$, preferably between 10 cm/cm$^2$ and 80 cm/cm$^2$,
- an adhesive layer (15, 15') at least partially covering the second face (5b) of the film (5) and adapted to hold the film (5) on the limb of the individual.

2. The holding device (1, 1') according to claim 1, wherein the wall (10) of each protrusion (7) extends around a central axis (A) substantially normal to the base surface (6), the length of the free edge (8) of each protrusion (7) defining a perimeter.

3. The holding device (1, 1') according to any one of claims 1 and 2, wherein the protrusions (7) are integrally formed in one-piece with the base surface (6).

4. The holding device (1, 1') according to any one of claims 1 to 3, wherein the film (5) includes a plurality of perforations (9) made throughout the film (5) from the second face (5b) so as to obtain local deformations of the film (5) on the first face (5a), each local deformation surrounding one of the perforations (9) and forming the wall (10) of one of the protrusions (7).

5. The holding device (1, 1') according to any one of claims 1 to 4, wherein the protrusions (7) are arranged according to a network having a density comprised between 15 protrusions/cm$^2$ and 700 protrusions/cm$^2$, preferably between 80 protrusions/cm$^2$ and 300 protrusions/cm$^2$.

6. The holding device (1, 1') according to any one of claims 1 to 5, wherein the second face (5b) of the film (5) has a smooth surface.

7. The holding device (1, 1') according to any one of claims 1 to 6, wherein the film (5) is made of a thermoplastic polymer, in particular of a polyolefin-based polymer, such as polyethylene, a copolymer of polyethylene and vinyl acetate, a copolymer of polyethylene and butyl acrylate or plasticised polyvinyl chloride, a polyester polymer or copolymer of, a polyamide polymer or copolymer of, a polyurethane polymer or copolymer or a polycarbonate polymer.

8. The holding device (1, 1') according to claim 7, wherein the film (5) has a basis weight comprised between 10 g/m$^2$ and 120 g/m$^2$, preferably between 20 g/m$^2$ and 60 g/m$^2$

9. The holding device (1, 1') according to any one of claims 1 to 8, wherein the adhesive layer (15, 15') has a tangential shear strength t of at least 10 hours according to a creep test in which a first portion (1a) of the holding device (1) is bonded via the adhesive layer (15, 15') to a plate (20) inclined at an angle of 2°, with respect to a vertical direction (V), leaving a second portion (1b) of the holding device (1) free, a weight (21) hanging from the second portion (1b) of the holding device (1), with: $t = (L^2 . l . \eta)/2.e.M.g$

where L is a length of the adhesive layer (15, 15') of the first portion (1a) of the holding device (1, 1'),
1 is a width of the adhesive layer (15, 15') of the first portion (1a) of the holding device (1, 1'),
$\eta$ is a viscosity of the adhesive layer (15, 15'),
e is a thickness of the adhesive layer (15, 15'),
M is a mass of the weight (21), M being selected according to a tangential stress exerted by the retention garment,
g = 9.81 m/s$^2$.

10. The holding device (1, 1') according to any one of claims 1 to 8, wherein the adhesive layer (15, 15') consists of a pressure-sensitive adhesive substance and in particular an adhesive substance selected from among:

- acrylic adhesives based on acrylate polymers or copolymers of acrylic acid and esters of acrylic acid,
- adhesives based on polyisobutylene with high molecular weight,
- adhesives based on natural or synthetic polyisoprene,
- adhesives based on siloxane polymers or copolymers and tackifier resins or silicone gels based on crosslinked polyxilosanes,
- adhesives based on polyurethane or polyurethane gels,

- adhesives based on triblock copolymers, plasticisers and tackifier resins, in particular triblock polymers of the styrene-central sequence-styrene type wherein the central sequence is unsaturated, for example isoprene or butadiene, or saturated, for example ethylene-butylene or ethylene-propylene, possibly mixed with diblock copolymers like, for example, styrene-isoprene or styrene-butadiene copolymers.

11. The holding device (1) according to claim 10, wherein the adhesive layer (15) includes one single elementary adhesive layer (16), the elementary adhesive layer (16) having in particular a basis weight of at least 50 g/m$^2$, preferably comprised between 60 g/m$^2$ and 120 g/m$^2$.

12. The holding device (1) according to claim 11, wherein the elementary adhesive layer (16) has an adhesive force comprised between 0.5 N/cm and 5 N/cm measured by a peel force test in which the adhesive force at an angle of 90° for detaching the adhesive layer (15) off a reference surface selected from among the second face (5b) of the film (5) and a skin surface is measured.

13. The holding device (1') according to claim 10, wherein the adhesive layer (15') comprises:

   - a first elementary adhesive layer (16a) intended to be brought into contact with the limb, the first elementary adhesive layer (16a) having in particular a basis weight comprised between 25 g/m$^2$ and 120 g/m$^2$, preferably between 40 g/m$^2$ and 80 g/m$^2$,
   - a core (17) having a first surface bearing the first elementary adhesive layer (16a), and a second surface opposite to the first surface,
   - a second elementary adhesive layer (16b) for fastening the second surface of the core (17) to the second face (5b) of the film (5), the second elementary adhesive layer (16b) having in particular a basis weight comprised between 20 g/m$^2$ and 120 g/m$^2$, preferably between 30 g/m$^2$ and 50 g/m$^2$.

14. The holding device (1') according to claim 13, wherein the first elementary adhesive layer (16a) has an adhesive force comprised between 0, 5 N/cm and 5 N/cm and the second elementary adhesive layer (16b) has an adhesive force comprised between 0.5 N/cm and 10 N/cm, the adhesive force of each of the elementary adhesive layers selected from among the first (16a) and second (16b) elementary adhesive layers being measured by a peel test in which the adhesive force at an angle of 90° for detaching said elementary adhesive layer off a reference surface selected from among the second face (5b) of the film (5) and a skin surface is measured.

15. A retention system comprising a retention garment intended to be placed on a limb of a patient, such as a textile retention band (2), in particular having a short elongation, intended to be wound on the limb, and a holding device (1) according to any one of claims 1 to 14.

FIG. 1

FIG. 2

FIG. 3

FIG. 4

FIG. 5

FIG. 6

FIG. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012140558 A
- WO 201594385 A
- WO 2016177829 A
- US 6194629 B
- WO 2009138672 A
- EP 281057 A
- DE 102006022971
- WO 2017089731 A
- WO 2009138642 A
- WO 2007113340 A